# EUROPÄISCHE PATENTSCHRIFT

(11) **EP 1 427 330 B1**
(45) Veröffentlichungstag und Bekanntmachung des Hinweises auf die Patenterteilung: **18.08.2010**
(21) Anmeldenummer: 02738415.5
(22) Anmeldetag: 11.06.2002
(51) Int. Cl.: A61B 5/0402, A61B 5/0404, A61B 5/0428

(54) **VERFAHREN UND GERÄT FÜR DIE ELEKTROKARDIOLOGISCHE SELBSTUNTERSUCHUNG**
METHOD AND APPARATUS FOR ELECTROCARDIOLOGIC SELF-EXAMINATION
PROCEDE ET DISPOSITIF DESTINES A UN AUTO-EXAMEN ELECTROCARDIOLOGIQUE

(30) Priorität: 12.06.2001 HU 0102418
(43) Veröffentlichungstag der Anmeldung: 16.06.2004
(73) Patentinhaber: Wolf, Tamás, 2000 Szentendre (HU); Bukosza, István, 1031 Budapest (HU)
(72) Erfinder: Wolf, Tamás, 2000 Szentendre (HU); Bukosza, István, 1031 Budapest (HU)
(74) Vertreter: Mak, Andras
(86) Internationale Anmeldenummer: PCT/HU2002/000053
(87) Internationale Veröffentlichungsnummer: WO 2002/100264

(56) Entgegenhaltungen:
- EP-A- 1 095 612
- WO-A-99/08592
- US-A- 6 038 469

## Beschreibung

### Stand der Technik.

Die vorliegende Erfindung bezieht sich auf ein Verfahren sowie auf eine Einrichtung zur elektrokardiologischen Selbstuntersuchung. Die meist gebräuchlichen verbreiteten intelligenten EKG-Einrichtungen - welche auf Grund der gemessenen Kurven einen EKG-Befund liefern - sind für Ärzte bestimmt, da die gelieferten Informationen für den untersuchten Patienten nicht verständlich sind. Die für den persönlichen Gebrauch bestimmten EKG-Einrichtungen sind im allgemeinen so ausgeführt, dass sie in kritischen Lagen die Daten des Patienten an eine Auswertungszentrale senden, wo eine Wertung und Analyse erfolgt, worauf der Patient über seinen Zustand und über die zu treffenden Massnahmen benachrichtigt wird.

Der Nachteil des Systems, - welches auf telefonischer Verständigung beruht - besteht darin, dass es kompliziert ist und nur gegen beträchtliche Monatsgebühren in Anspruch genommen werden kann.

Ebenfalls durch telefonische Verständigung wird ein anderes System betrieben, bei welchem die an eine Zentrale gesandten EKG - Daten ohne jede operative Bearbeitung als FAX zur Verfügung stehen. Die Bewertung der eingesandten EKG - Daten wird vom Arzt der Empfangsstation durchgeführt.

Die neueste Generation der Einrichtungen die zur EKG Untersuchungen durch den Patienten in seinem Heim geeignet sein sollen (Ung.PA WO 99/08592) beinhalten eine solche Einrichtung, welche ohne Mitwirkung des Arztes gebraucht werden kann. Die Einrichtung ist so ausgeführt, dass bei einer Änderung der EKG -Parameter der Patient durch ein Alarmzeichen zur Aerztekonsultation aufgefordert wird. Diese automatische EKG-Einrichtung kann ohne Mitwirkung des Arztes gebraucht werden. Zur Auslösung des Alarmzeichens wird ein Algorythmus gebraucht, welches durch einfache mathematische Formeln bestimmt werden kann und keinerlei ärztlichen Ueberlegungen bedarf. Der Nachteil dieser Einrichtung besteht jedoch darin, dass sie bei jeder Änderung der ausgewählten EKG-Zeichen Alarm gibt, ohne dabei den kardiologischen Zustand des Patienten zu berücksichtigen. Falls jedoch die den Alarm auslösenden Parameteränderungen für den Zustand des Patienten unwesentlich sind, so wurde der Patient unnötig zum Arzt geschickt. Schliesslich ist aus dem U.S.Patent 5.501.229 ein Instrument zur kontinuierlichen Überwachung eines Herzkranken bekannt geworden, welches Instrument in periodisch wiederholten Zyklen den EKG- Kurvenverlauf des Patienten überwacht .Es wird ein Alarm generiert, wenn der Patient in lebensbedrohliche Lage geriet. Das Instrument kann nur unter klinischen Bedingung und vom Fachpersonal bedient gebraucht werden. Auch die gelieferten Resultate können nur vom Arzt interpretiert werden.

Das Dokument EP-1095612-A offenbart ein Verfahren zur kardiologischen Selbstuntersuchung mittels einer automatischen EKG-Diagnostiziereinrichtung und zum Generieren einer Entscheidungshilfe für den Kranken, wobei eine für den Grundzustand des Kranken charakteristische Grunddiagnose in der Diagnostiziereinrichtung gespeichert wird. Die Reihenfolge der aufgesetzten Elektroden wird nicht kontrolliert.

Gegenüber diesen bekannten Einrichtungen wird ein Verfahren und eine Einrichtung zur Ausübung des Verfahrens vorgeschlagen, welche in Abhängigkeit der Zustandsänderung des Patienten, aus mehreren verschiedenen möglichen Alarmstufen ausgewählten Alarm gibt und auch bei einfachen, häuslichen Verhältnissen vom Kranken selbst eingesetzt und zuverlässig bedient werden kann.

### Zusammenfassung der Erfindung.

Nach dem vorgeschlagenen Verfahren wird mit Hilfe der Einrichtung auf der Grundlage einer EKG-Messung, welche der Patient selbst durchführt, eine EKG - Diagnose erstellt und mit einer vom Arzt früher bestimmten und in der Einrichtung vorhandenen Grunddiagnose (Etalon) verglichen, worauf das Resultat in einer für den Patienten verständlichen Form vorgelegt wird. Die Reihenfolge der Elektroden wird kontrolliert

Das Verfahren zur elektrokardiologischen Selbstuntersuchung und die Einrichtung zur Ausübung des Verfahrens sind in den unabhängigen Ansprüchen definiert. Einzelheiten und besondere Ausführungsformen des Verfahrens und der Einrichtung sind in den zugeordneten weiteren Ansprüchen enthalten..

Gemäss einer zur Ausübung des Verfahrens geeigneten Einrichtung - welche ein automatisches EKG-Diagnostiziergerät umfasst - kann auf Grund einer durch den Patienten selbst durchgeführten Messung eine Diagnose erstellt und diese im Vergleich mit einer Etalondiagnose bewertet werden. Es wird ferner erreicht, dass die Alarmgebung nicht in Funktion der Änderung von herausgegriffenen Parameter erfolgt, sondern es wird auf Grund der aktuellen EKG-Diagnose ein bestimmter Alarm oder eine Mitteilung generiert, welcher bzw. welche aus mehreren Möglichkeiten ausgewählt wurde. Die Wahl erfolgt in Abhängigkeit vom diagnostizieiten Zustand des Patienten. Die Grund - oder Etalondiagnose dient also nicht dazu den Patienten, bei Abweichungen der aktuell erstellten Diagnose von der Grunddiagnose, zum Arzt zu schicken, sondern im Gegenteil, es soll erreicht werden, dass die aktuelle Diagnose keinen, unnötigen Alarm auslöst, wenn in Vergleich mit der Grunddiagnose keine gravierend Verschlechterung im Zustand des Patienten erfolgte.

Das Vorgehen der Einrichtung nach dem Verfahren ist sogar lernfähig, da der Arzt des Patienten die Einrichtung zur Auslegung der zu Hause erzielten Resultate programmiert, so dass. ein von der Norm abweichendes EKG-Diagramm keinen unnötigen Alarm auslösen wird, wenn das Grunddiagramm verbietet einen nach der aktuellen krankhaften EKG-Diagnose sonst berechtigten Alarm auszulösen.

Beim vorgeschlagenen Verfahren findet bei jeder EKG-Messung eine differentialdiagnostische Analyse statt.. Gemäss der Diagnose wird entweder "roter Alarm" (sofortiger Arztbeistand notwendig) oder "gelber Alarm" (mit der Arztkonsultation kann zugewartet werden) oder "keine Änderung". generiert . Voraussetzung zum "roten Alarm" ist wenn die vom Patienten durchgeführte und von der Einrichtung erarbeitete Diagnose solche krankhafte Änderungen aufweist, die in der Grunddiagnose nicht vorhanden waren und den sofortigen Einsatz des Arztes verlangen. Gelber Alarm entsteht dann, wenn der Zustand des Patienten zwar krankhaft ist, aber unter Berücksichtigung der die Grundkrankheit spiegelnden Diagnose, keine solche Verschlimmerung bedeutet, welche einen sofortigen ärztlichen Beistand notwendig machen würde. Bei "Keine Änderung" ist keine Arztkonsultation nötig. In allen drei Fällen werden das EKG-Diagramm und die Grunddiagnose in der Einrichtung gespeichert und bleibt so für den Arzt bei der Bestimmung der Therapie verfügbar. Selbstverständlich könnten auch noch mehr als drei Alarmstufen vorhanden sein.

Da der Patient selbst die EKG-Messung in seinem Heim durchführt, muss das übliche laboratoriummässige Vorgehen vereinfacht werden. Allgemein wird eine EKG-Einrichtung mit zwölf Ableitungen und zehn Messelektroden verwendet, von welchen sechs Messelektroden im Brustbereich und vier an den Gliedmassen plaziert werden müssen.

Die richtige Ortung der sechs Messelektroden für den Brustbereich bildet für den Laien eine unmögliche Aufgabe. Bei wiederholten Messungen würden ungenau aufgesetzte Elektroden zu falschen Messresultaten führen, wodurch Fehlalarme ausgelöst werden könnten. Um diese Schwierigkeiten zu begegnen wird vorgeschlagen; neben den vier Elektroden zu den Gliedmassen, eine reduzierte Anzahl z.B. eins, zwei oder drei Messelektroden im Brustbereich zu verwenden. Grundsätzlich werden im Brustbereich zwei Elektroden V2 und V5 verwendet, welche mit Sicherheit richtig aufgesetzt werden können und eine genügend exakte Diagnose sichern. Die richtige Plazierung der Elektroden wird den Patienten vom Arzt gezeigt bei der Vorführung der Einrichtung.. Die gewählten richtigen Stellen können am Körper z.B. mit einem geeigneten Stift bezeichnet werden. Das Verfahren gibt jedoch die Möglichkeit, nach Entscheid des behandelnden Arztes, jede beliebige Kombination zu wählen. Man kann sowohl. eine Elektrode (z.B. V3 oder V4) oder zwei (z.B.V2 und V5 oder V3 und V6) oder drei (z.B. V2,V4,V6 oder V1,V3, V5) für den Brustbereich wählen. Bei der Auswahl der Elektroden wird die Grundkrankheit des Patienten und seine Lernfähigkeit berücksichtigt. Sofern entsprechend geschulte äussere Hilfe zur Verfügung steht, so kann die Einrichtung auch an Hand von sechs Elektroden das Diagramm analysieren.

Da die EKG-Messung nicht in ärztlicher Umgebung sondern beim Patienten zu Hause stattfindet, ist es erforderlich die sonst noch auftretenden Schwierigkeiten zu lösen. Diese ergeben sich daraus, dass der Messbeginn nach Aufsetzen der Elektroden verzögert erfolgen muss und dass die Qualität der gelieferten Mess - Signale und die Reihenfolge der Ableitungen überwacht werden muss. Es wird deshalb im Rahmen der Erfindung ein Verfahren zur Verzögerung des Messbeginns, eine automatische Kontrolle der Signalqualität und eine Kontrolle der Reihenfolge der Ableitungen vorgeschlagen. Diese Hilfsmittel können für den Patienten bei der Bedienung der Einrichtung die Anwesenheit vom Fachpersonal ersetzen.

Zusammenfassend kann man sagen, dass das Verfahren und die Einrichtung ein Hilfsmittel für den Patienten bedeutet, welches gekauft und auch durch Laien bedient werden kann. Ferner bildet das Resultat der Untersuchung eine leicht verständliche Information für den Patienten und eine Hilfe für den Arzt, um mit den notwendigen Massnahmen unverzüglich beginnen zu können.

Beschreibung der Erfindung.

Auf beiliegender Zeichnung ist ein Ausführungsbeispiel der Einrichtung schematisch dargestellt, auf Grund welcher auch das Verfahren näher erläutert wird.

Es zeigen:
Fig.1 ein Blockschema der Einrichtung und
Fig.2 ein Arbeitsschema, nach welcher, die Geräuschkontrolle, die Kontrolle der Amplituden der Signale und sowie die Urberwachung der Reihenfolge der Anordnung der Elektroden erfolgt..

Die Einrichtung kann nur dann dauernd von Patienten gebraucht werden, wenn die Einrichtung käuflich erworben werden kann. Die vorgeschlagene Einrichtung hat deshalb solche Vereinfachungen, welche eine kleine handliche Ausführung gestatten und einen wesentlichen Preisnachlass gegenüber den bekannten professionellen Einrichtungen ermöglichen. Dabei stehen sämtliche wichtige Informationen zur Beurteilung der EKG-Signale unverändert zur Verfügung.

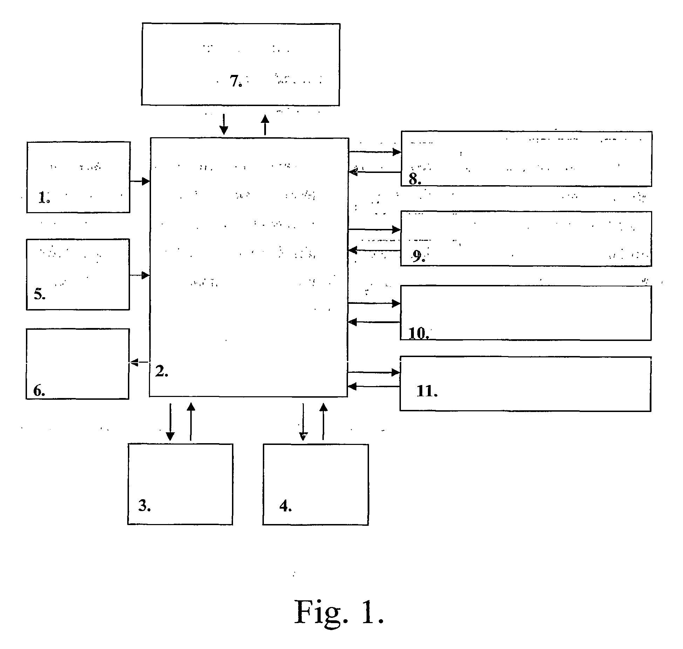

Die Einrichtung weist einen Messdatensammler mit Mikroprozessoren auf. Wie aus Fig. 1 ersichtlich ist steht ein Achtkanal - Analogverstärker 1 mit einer Mess- Steuer und Diagnostiziereinheit 2 in Verbindung. Die Datenverarbeitung erfolgt nach einem Programm das in einer FLASH-Memorie 3 gespeichert ist, während die vorübergehende Speicherung der digitalisierten EKG-Daten in einer RAM-Memorie 4 erfolgt. Als . Verbindung mit dem Anwender ist ein alfanumerisches Anzeigegerät 6 und eine Schalterreihe 5 mit vier Druckknöpfen vorhanden. Die Verbindung mit einem Computerrechner erfolgt über eine Infrarot-Komunikationseinheit 7. Zur Programmierung, und zur Speicherung und Aufzeichnung der Messwerte dient ein Computerrechner. Die erwähnten Elemente 1 - 7 bilden Bestandteile einer allgemein gebräuchlichen, intelligenten EKG-Einrichtung, an welche - im Sinne der Erfindung - die Teile 8,9,10 und 11 angeschlossen sind. 8 ist eine Verzögerungseinheit zum Verzögern des Messbeginns, 9 eine Signalqualitäts - Kontrolleinheit, 10, überwacht die Reihenfolge der Ableitungen und 11 ist die Einheit zur Analyse der Messdaten.

Die Vereinfachung des Ableitungsystems.

Wie bereits erwähnt ist beim häuslichen Gebrauch durch Laien die allgemein übliche Verwendung von zwölf Ableitungen nicht vorteilhaft, da das fehlerlose Anbringen der gebräuchlichen sechs Brust - Elektroden auch vom Fachmann grosse Aufmerksamkeit verlangt. Um die EKG-Messung zu vereinfachen, werden bei der Messung an Stelle der üblichen zwölf Ableitungen und zehn Elektroden - vier an den Gliedmassen und sechs Brustelektroden - nach der Erfindung insgesamt nur fünf, sechs oder sieben Elektroden gebraucht. Dem Entscheid des Arztes entsprechend werden neben den vier an den Gliedmassen zu plazierenden Elektroden, deren richtige Anordnung keinerlei Schwierigkeiten bereitet, nur noch eins, zwei oder drei Elektroden im Brustbereich verwendet. In den meisten Fällen genügen die Signale von zwei Brustelektroden und zwar die Signale der Elektroden V2 und V5 um eine genügende Genauigkeit der Diagnose zu erreichen. Der EKG - Messvorgang ist in dieser Weise für den Patienten wesentlich vereinfacht und der reduzierte EKG - Befund genügt für den Patienten um zu entscheiden ob ein Arztbesuch nötig ist oder nicht. Das Programmsystem der Einrichtung gibt u.a. dem Benützer eine Anweisung, verschiedene Aufgaben zu lösen, wobei jede Aufgabe auf dem Bildschirm des Anzeigegerätes erscheint. Durch die Betätigung des zutreffenden Knopfes der Schalterreihe 5 schaltet das System weiter. Jede Anordnung oder Frage, welche am Anzeigegerät erscheint, kann durch die Betätigung des JA oder NEIN Knopfes beantwortet oder bestätigt werden.. Die Erledigung jeder Aufgabe - z.B. das Aufsetzen der Elektroden - wird auch durch Tonzeichen bestätigt. Wird die Aufgabe nicht richtig gelöst, so erfolgt eine abweichende Tongebung.

Die automatische Verzögerung des Messbeginns.

Da die EKG-Selbstmessung ohne Fachassistenz erfolgt, sorgt die in Fig.1 gezeigte Messverzögerungseinheit 8 dafür, dass nachdem der Kranke Befehl zur Messung gegeben hat, die Messdaten erst nach einer Verzögerung gesammelt werden. Diese Verzögerung sichert, dass die notwendige Verbindung zwischen der trockenen Haut und den nassen Elektroden hergestellt ist, sowie dass der Kranke vor Einsetzen.der Messung sich entspannen kann, wodurch die Muskelgeräusche gemindert werden. Bei einem Messvorgang im Laboratorium bzw. in der Arztpraxis wird diese Verzögerung durch den Assistenten manuell eingestellt, indem das EKG-Gerät beobachtet wird und nach dem Aufsetzen der Elektroden einige Sekunden gewartet wird, bevor die Messung einsetzt:

Die automatische Kontrolle der EKG -Signalqualität.

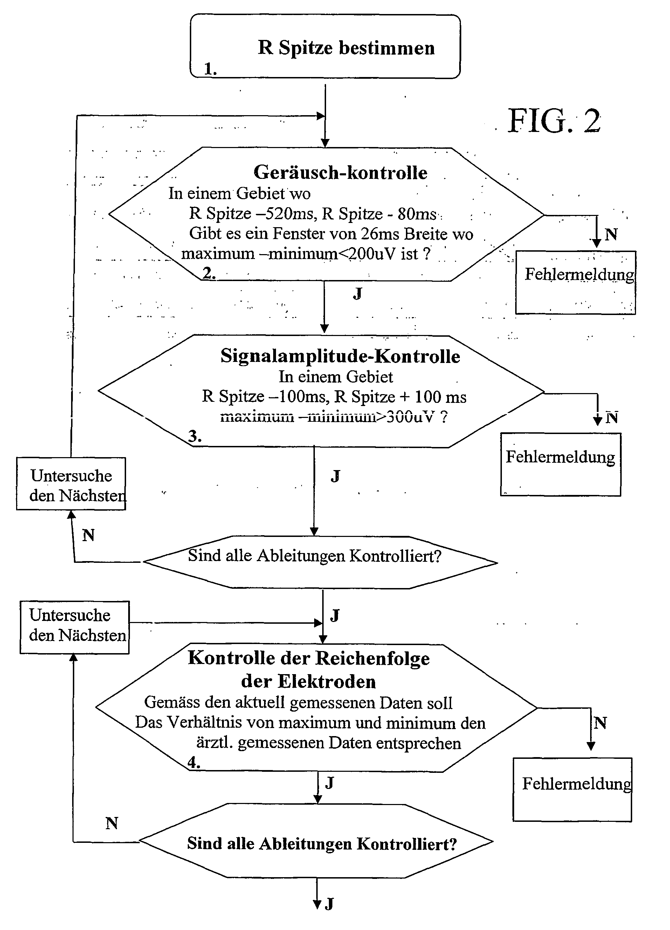

Erst die vorgeschlagene automatische Kontrolle der EKG-Signalqualität erlaubt, dass der Patient selbst seine EKG-Kurve bestimmen kann. Das Programm der entsprechenden Kontrolleinrichtung ist so gestaltet, dass als Resultat der durchgeführten Kontrollen, der Patient eine Warnung erhält, wenn er die Elektroden nicht richtig aufgesetzt hat. Gemäss dem vorgeschlagenen Verfahren wird das richtige Aufsetzen der Elektroden wie folgt kontrolliert : (Das Vorgehen wird an Hand der Fig. 2 erläutert.) Nach dem die Spitze R ermittelt wurde (S.Pos.1 der Fig.2) wird für jede Ableitung durch ein Algorythmus der Geräuschpegel und die nützliche Amplitude des Signals kontrolliert. Beim werten des Geräusches benützt das Verfahren diejenige Eigenschaft des EKG-Signals, dass die Signalstrecke vor den grössten Ausschlag (R Spitze) immer einen Teil ohne Signal - die sog. Basislinie - aufweist. Die Kontrolle des Geräusches in einem Gebiet (R Spitze - 520ms, R Spitze - 80ms) vor der Spitze R wird in einem 26 ms breiten gleitenden Fenster durchgeführt. Falls eine Fensterstellung vorhanden ist, in welcher max - min < 200uV ist, so ist das Geräusch der fraglichen Ableitung annehmbar. (Pos.2 der Fig.2).

Die Signalamplitudenkontrolle wird in einem Gebiet R Spitze -100ms, R Spitze +100ms durchgeführt. (S.Pos.3 der Fig.2). Falls hier maximum - minimum >300uV, so ist die Signalamplitude annehmbar. Falls grösseres Geräusch oder kleinere Signalamplitude als erlaubt gemessen wird, so erfolgt eine Fehlermeldung, welche die Berichtigung der Stellung der Elektrode verlangt. Falls beim wiederholten Messen das Geräusch immer noch zu hoch ist, so wird eine weitere Fehlermeldung erscheinen. Eine zu kleine Amplitude wird aber beim dritten Versuch angenommen, in der Annahme, dass die kleine Amplitude eine Eigenheit des EKG Signals des Patienten ist.

Die automatische Kontrolle der Reihenfolge der EKG Elektroden.

Hier wird auf Pos.4 der Fig.2 verwiesen. Die Kontrolle erfolgt nach dem Verfahren auf Grund der Eigenart der Randwerte von einzelnen Ableitungen. Die Randwerte der einzelnen Ableitungen des Etalon EKG und des aktuellen. EKG, sowie die Eigenart von Maximum und Minimum, ändern sich beim stabilen kardiologischen Zustand nicht. Bei der Bestimmung der Etalon EKG-Diagnose bestimmt das Verfahren für jede der Ableitungen im Bereiche der R Spitze die maximalen und minimalen Amplitudenwerte d.h. den grössten maximalen und minimalen Ausschlag. Die für den Ausschlag jeder der Ableitungen charakteristische Konstante wird durch Dividieren der absolut grössten und absolut kleinsten Amplitudenwerte berechnet: KI, KII, KVI, KV2, KV3, KV4, KV5, KV6. Ki hat einen ständigen Wert von 0 wenn der Quotient zwischen -1,5 und + 1,5 liegt, Ki = +1 wenn der Quotient grösser ist als 1,5 , und Ki = -1 wenn K <-1,5 ist. Die Ki Werte werden in der Einrichtung gespeichert. Bei der EKG Selbst - Messung wird gemäss dem Verfahren überprüft ob die Eigenart der Randwerte gegenüber dem Etalon in den angewendeten Ableitungen unverändert geblieben sind. Falls in einer Ableitung K1 = 0, so erfolgt in dieser Ableitung keine Kontrolle, da Maximum und Minimum sich nicht wesentlich unterscheiden und als Folge einer zufälligen Änderung des EKG-Signals das Quotient sich ändern könne, ohne dass die Elektrode falsch aufgesetzt wäre. In den Ableitungen, wo Ki = +1 bzw. Ki = -1 wird gemäss dem Verfahren untersucht ob bei der EKG Messung, das zur gegebenen Ableitung berechnete K-Wert sein Vorzeichen beibehalten hat oder nicht. Sofern das Vorzeichen sich in einer der Ableitungen geändert hat, so wird mit einer Fehlermeldung um Kontrolle gebeten. Falls nach der Kontrolle der Patient die Anordnung der Elektroden als richtig fmdet, so wird die beanstandete Messung angenommen und die Diagnose erstellt. Wenn der Patient die Anordnung der Elektroden verbessert hat, so wird eine neue Messung eingeleitet.

Das diagnostische Analysenverfahren.

Zweck des diagnostischen Analysenverfarens ist es einen solchen Entscheid herbeizuführen, welcher dem Patienten klar zu erkennen gibt, ob sein aktueller Zustand den Arzt zu konsultieren nötig macht bzw. ob er eventuell sofortige Hilfe braucht. Falls die obigen Fragen nur mit Ja oder Nein beantwortet werden, so wird der Patient in den meisten Fällen umsonst zum Arzt gehen, da er von der Schwere der festgestellten Anomalie keine Auskunft erhalten hat. Falls die angewendete Untersuchungsmethode nur:das Vorzeichen und die Grösse der Änderung eines EKG-Parameters feststellt und die Änderungen nicht in Funktion der Grund- EKG Diagnose betrachtet werden, so machen die daraus resultierenden zahlreichen falschen Entscheidungen und unnötigen Alarme solche Untersuchungsmethoden praktisch unbrauchbar.

Gemäss dem vorliegenden Verfahren wird deshalb die aktuelle Diagnose immer in Funktion der EKG - Grunddiagnose bewertet. Auf Grund der Bewertung wird ein dreistufiger Entscheid - "keine Änderung", "gelber Alarm" und "roter Alarm gefällt.. Der Entscheid nach Stufe 1 d.h. "keine Änderung" wird auch dann gefällt, wenn das aktuelle EKG Diagramm zwar krankhaft ist, aber auch das Grunddiagramm nicht normal war. Bloss eine Änderung der EKG Parameter bildet noch keinen genügenden Grund um den Kranken zum Arzt zu schicken, da z.B. bei chronischem Ischämie eine max. 50uV niedrige positive T-Welle und eine von der Null - Linie kaum abweichende negative T - Welle sich zwar mathematisch abweichen, aber bezüglich der EKG Diagnose als gleichwertig zu betrachten sind.

Gelber Alarm wird generiert, wenn eine schon bekannte EKG Abnormität - z.B. Ischämiesich in der schlechten Richtung geändert hat, z.B. wenn die ST - Abweichung grösser geworden ist. Roter Alarm wird entschieden, wenn die aktuellen EKG Abweichungen die notwendigen und hinreichenden Voraussetzungen eines krankhaften EKG Befundes erfüllen und dieser krankhafte EKG - Befund bei den Patienten früher noch nie vorgekommen ist..

Es ist wesentlich gemäss der Erfindung, dass die Einrichtung vom Patienten erst dann gebraucht werden kann, wenn diese vom Arzt programmiert wurde. Das Programmieren erfolgt mit Hilfe des Arzt-Computers. Bei der Programmierug macht der Arzt eine EKG - Messung mit der Einrichtung, wobei zwölf Ableitungen verwendet werden.

Anschliessend wird die Messung mit dem eingebauten Diagnostikprogramm analysiert. Die EKG - Diagnose kann visualisiert und kontrolliert werden. Die gut befundene oder korrigierte Diagnose wird in der Einrichtung gespeichert und dient für spätere Messungen als Etalonwert. Bei der Bestimmung dieser Etalondiagnose benützt der Arzt die gleiche Sammlung der Diagnosen - Kategorien, welche im Diagnosenprogramm der Einrichtung vorhanden ist. In dieser Weise kann das Resultat, welches der Patient bei der Selbstmessung erzielt, mit der Diagnose im Etalon verglichen werden. In dieser Phase hat der Arzt die Möglichkeit, die Alarmkriterien zu modifizieren und an den Patient speziell anzupassen. Gleichzeitig wird der Patient vom Arzt unterrichtet wie die Elektroden auf den Gliedmassen und im Brustbereich anzuordnen und festzumachen sind, wobei - wie früher schon erwahnt, -im Brustbereich meistens eine Elektrode, oder nötigenfalls zwei bis sechs Elektroden, zum Einsatz kommen sollten.

Die Einrichtung analysiert die Abweichungen der aktuellen Diagnose von der gespeicherten Grunddiagnose in gleicher Weise wie der Arzt es machen würde. Falls die Abweichungen unwesentlich sind, so gibt die Einrichtung an der Anzeigetafel bekannt: "Keine wesentliche Änderung". Wenn die Abweichungen wesentlich sind aber eine sofortige Visite beim Arzt nicht erforderlich erscheint, so erscheint an der Anzeigetafel: "Kleine Änderung am EKG, konsultieren Sie den Arzt". Wenn die Abweichung gravierend ist und eine sofortige Arztkonsultation verlangt, so wird mitgeteilt: "EKG geändert. Konsultieren Sie sofort den Arzt".

Aus den vorausgehenden Ausführungen ergibt sich, dass die Voraussetzung damit die Einrichtung arbeitet darin besteht, dass der Etalon des Patienten vom Arzt analysiert wird. Nachdem die Einrichtung gekauft wurde, wird die erste EKG -Messung vom behandelnden Arzt ausgeführt und das Resultat gespeichert. Diese Etalon - Diagnose bleibt solange in der Einrichtung bis der Zustand des Patienten sich so geändert hat, dass eine neue Etalon - Messung als notwendig erscheint, welche dann die alten Messresultate ersetzen wird. Der Patient nimmt also die so vorbereite Einrichtung mit nach Hause und kann mit der Selbstmessung beginnen. So kann der Zustand, wenn nötig täglich oder mehrmals täglich kontrolliert werden. Die Alarmmeldungen, können u.a. durch Rythmus, Depolarisation oder Repolarisation verursacht werden. Die verschiedensten Ursachen können in Tabellen zusammengefasst werden.

Ein wesentliches Merkmal der Erfindung bildet das Verfahren zur Analyse der Abweichungen der aktuellen Diagnose vom Etalon. Demgemäss werden nicht die Parameteränderungen, sondern es wird die aktuelle Diagnose selbst in Abhängigkeit von der Grunddiagnose ausgewertet. Bei der Auswertung bringt das Verfahren die Möglichkeit die Beschwerden des Patienten zu berücksichtigen, indem das human-diagnostische ; Verfahren simuliert wird. In ähnlicher Weise wie in der Arztpraxis werden in Frage/Antwort Form die Klagen des Patienten ermittelt und zwar durch Fragen die durch Ja oder Nein beantwortet werden können. Die Antworten werden gemäss dem. Verfahren ausgewertet und bei der Analyse der aktuellen EKG berücksichtigt. Es ist möglich, dass bei "Keine signifikante Änderung "oder bei "Kleine EKG Änderung" trotzdem Rotalarm ausgelöst wird, wenn dies durch die Klagen des Patienten als begründet erscheint.

Nach dem Verfahren wird auf Grund der EKG-Analyse nur dann Alarm ausgelöst, wenn die aktuelle Diagnose auf Grund des Etalons nicht aufgestellt werden konnte. Es ist wesentlich noch zu erwähnen, dass diejenigen verschiedenen Diagnosen, welche an einer "zufälligen" Änderung des EKG beruhen, aber aus kardiologischer Hinsicht und/oder therapischer Sicht keine geänderten Zustände spiegeln, vom Verfahren als gleichwertig betrachtet werden. Die Aehnlichkeit oder Wechselwirkung zwischen den in Betracht gezogenen Diagnosen wird im Sinne des Verfahrens auf Grund der sog.

Wechselwirkungsvektoren untersucht. Jeder Krankheitskategorie entspricht ein Wechselwirkungsvektor, welcher ein einziges nullwertiges Element besitzt, wenn die gegebene Kategorie sich von jeder anderen Diagnosenkategorie abweicht bzw. die Ordnungszahl der ähnlichen Diagnosenklassen enthält. Die Wechselwirkungsvektoren der Krankheitsklassen können ebenfalls nach Rythmus, Depolarisation und Repolarisation tabellarisch zusammengefasst werden.

Anschliessend werden Beispiele von Alarmmeldungen aufgezählt:
Alarm auf Grund von Rythmus Analyse:
   Gelber Alarm: Krankhaft gedehnter R-R Abstand (RRmax >1,5 sec)
   Roter Alarm: Gefährlich gedehnter R-R Abstand (RRmax>2sec)
   Gelber Alarm : Krankhaft gedehnter PQ(PQ>200ms und 1,125<dPQ>1,25)
   Roter Alarm Neuer A - V Block III.Grad
   Roter Alarm: sehr frühe Kammer-Extrasystole (auf der. T-Welle)
Alarm auf Grund von Depolarisations-Analyse
   Gelber Alarm: Krankhafte Q-Welle in Inferiorableitungen, während in der Grunddiagnose entweder Q(II) oder QIII oder Q(aVF) pathologisch war.
   Gelber Alarm Wahrscheinlichkeit einer Hypertrophie der linken Kammer.
   Roter Alarm: Neue hypertrophie der rechten Kammer
   Roter Alarm: Akuter lateraler M1 Infarktverdacht, während auf der Grunddiagnose Q(I) und Q(aVL) und Q(V5 normal waren.
Alarm auf Grund von Repolarisations-Analyse:
   Gelber Alarm: Neue inferior Ischämie mit minor ST-Elevation
   Roter Alarm : Gefährliche inferior Ischämie mit wesentlicher ST Abweichung.
   Roter Alarm : Inferior Ischämie mit gefährlich gewachsener T-Welle

Die obigen Aufzählungen wurden nur als Beispiele erwähnt. Bei der Programmierung der Diagnosiziereinheit müssen alle Alarmauslösefaktoren berücksichtigt werden, die für einen umfassenden Einsatz der Einrichtung notwendig sind.

Darüber hinaus besteht noch die Möglichkeit die aktuellen Beschwerden des Kranken zu berücksichtigen. Im Rahmen der Selbstuntersuchung wird in diesem Falle eine Befragung durch die Einrichtung erfolgen, wobei bei einer auf ausgewählten Fragen erteilten JA-Antwort ein sonst begründeter gelber Alarm in roten Alarm geändert wird. Solche Fragen sind z.B. Haben Sie Schmerzen in der Halsgegend? Haben Sie kalte Schweissausbrüche? Wenn diese od. ähnliche Fragen mit Ja beantwortet werden, so besteht Verdacht, dass eine schwere Verschlechterung des kardiologischen Zustandes erfolgte, welche im momentanen EKG-Befund noch nicht erschienen sind.

### Liste der Bezugszeichen:

| | |
|---|---|
| 1 | Achtkanal-Verstärker |
| 2 | Messsteuer und Diagnostiziereinheit |
| 3 | FLASH Memorie |
| 4 | RAM Memorie. |
| 5 | Schalterreihe |
| 6 | Anzeigegerät |
| 7 | Infra - Kommunikationseinheit |
| 8 | Verzögerungseinheit zum verzögern des Messbeginns |
| 9 | Signalqualitäts-Kontrolleinheit |
| 10 | Einheit zur Kontrolle der Reihenfolge der Ableitungen |
| 11 | Analysiereinheit |

## Patentansprüche

1. Verfahren zur elektrokardiologischen Selbstuntersuchung, mittels einer automatischen EKG-Diagnostiziereinrichtung, welche mit Mitteln zum Speichern mindestens einer Diagnose und zum Generieren einer Entscheidungshilfe für den Kranken ausgerüstet ist, mit den nachfolgenden Schritten:
- es wird eine für den Grundzustand des Kranken charakteristische Grunddiagnose in der Diagnostiziereinrichtung gespeichert;
- es wird die Reihenfolge der aufgesetzten Elektroden vor einer Selbstmessung kontrolliert und bei Abweichung eine Fehlermeldung ausgegeben;
- auf Grund einer Selbstmessung des Kranken wird eine aktuelle Diagnose erstellt und in der Einrichtung gespeichert;
- die aktuelle Diagnose wird mit der Grunddiagnose verglichen;
- auf Grund des Vergleiches wird die Entscheidungshilfe in Funktion gesetzt.

2. Verfahren nach Anspruch 1, **dadurch gekennzeichnet, dass** die für den Grundzustand des Kranken charakteristische und mit der späteren aktuellen Diagnose zu vergleichende Grunddiagnose, eine über den Kranken in der Arztpraxis früher erstellte und in der Einrichtung gespeicherte Diagnose ist.

3. Verfahren nach den Ansprüchen 1 und 2, **dadurch gekennzeichnet, dass** die für den Grundzustand des Kranken charakteristische und mit der späteren aktuellen Diagnose zu vergleichende Grunddiagnose, eine aus dem Diagnosenvorrat der Diagnostiziereinrichtung ausgewählte, für die Krankheit des Kranken charakteristische und in der Einrichtung gespeicherte Diagnose ist.

4. Verfahren nach den Ansprüchen 1 - 3, **dadurch gekennzeichnet, dass** nach Einleitung des EKG Messbeginns durch den Kranken, die automatische Lieferung der Messdaten durch die EKG Diagnostiziereinrichtung verzögert in Gang gesetzt wird.

5. Verfahren nach den Ansprüchen 1 -4, **dadurch gekennzeichnet, dass** im Rahmen der Selbstmessung durch den Kranken, die richtige Anordnung der Elektroden der EKG-Diagnostiziereinrichtung am zu untersuchenden Körper, durch die Kontrolle der Signalqualität des EKG-Zeichens überprüft wird, in der Weise, dass die innerhalb des signallosen Gebietes der Kurve gemessene Differenz der auftretenden maximal und minimal Amplituden als Geräusch betrachtet und je nach dessen Verhältnis zu einem Schwellenwert, das Signal angenommen oder zurückgewiesen wird.

6. Verfahren nach den Ansprüchen 1 - 5, **dadurch gekennzeichnet, dass** im Rahmen der Selbstmessung durch den Kranken, zur Kontrolle der richtigen Reihenfolge der Elektroden der Diagnostiziereinrichtung, bei jeder Ableitung der durch den Arzt durchgeführten und zu Hause wiederholten Messung, die Eigenart der Randwerte miteinander verglichen werden und im Falle einer Abweichung ein Warnzeichen gegeben wird.

7. Einrichtung zur Ausübung des Verfahrens nach Anspruch 1, mit Hilfe einer automatischen EKG, Diagnostiziereinrichtung, die eine Mess-, Steuer-, und Diagnostiziereinheit (2) besitzt, welche zur Speicherung einer in der Arztpraxis erstellten Grunddiagnose geeignet ist, wobei die EKG-Diagnostiziereinrichtung ein Analysiergerät (11) aufweist, das die durch den Kranken durch Selbstmessung eingeleitete Diagnose mit der Grunddiagnose vergleicht und für den Patienten eine Entscheidungshilfe liefert, **dadurch gekennzeichnet, dass**, an die Diagnostiziereinheit (2) eine Kontrolleinheit zur Kontrolle der Reihenfolge der Ableitungen angeschlossen ist.

8. Einrichtung nach Anspruch 7, **dadurch gekennzeichnet, dass** zur Mess-, Steuer- und Diagnostiziereinheit (2) eine Verzögerungseinheit (8) für den Messbeginn angeschlossen ist.

9. Einrichtung nach den Ansprüchen 7 oder 8, **dadurch gekennzeichnet, dass** an die Diagnostiziereinheit (2) eine Signalqualitäts - Kontrolleinheit (9) angeschlossen ist.

## Claims

1. Method for electrocardiologic self-examination by means of an automatic ECG diagnosis device which is equipped with a device for storing at least one diagnosis and generating a decision aid for the patient, having the following steps:
- a characteristic initial diagnosis for the initial condition of the patient is stored in the diagnosis device;
- the sequence of applied electrodes is checked before self-measurement and an error message is emitted if there is a discrepancy;
- a current diagnosis is generated and stored in the device on the basis of self-measurement by the patient;
- the current diagnosis is compared with the initial diagnosis;
- the decision aid is put into operation on the basis of the comparison.

2. Method according to claim 1, **characterised in that** the initial diagnosis, which is characteristic of the initial condition of the patient and is to be compared with the later current diagnosis, is a diagnosis generated for the patient previously in the surgery and stored in the device.

3. Method according to claims 1 and 2, **characterised in that** the initial diagnosis, which is characteristic of the initial condition of the patient and is to be compared with the later current diagnosis, is a diagnosis characteristic of the patient's illness which is selected from a stock of diagnoses in the diagnosis device and stored in the device.

4. Method according to claim 1 to 3, **characterised in that** after the patient triggers the ECG measurement initiation, the automatic delivery of the measurement data by the ECG diagnosis device is started up after a delay.

5. Method according to claims 1 to 4, **characterised in that** in the self-measurement by the patient, the correct arrangement of the electrodes of the ECG diagnosis device on the body to be examined is monitored by checking the signal quality of the ECG signal, in such a way that the difference, measured within the signal-free region of the curve, between the maximum and minimum amplitudes which occur is treated as noise, and depending on the ratio thereof to a threshold the signal is accepted or rejected.

6. Method according to claims 1 to 5, **characterised in that** in the self-measurement by the patient, to check the correct sequence of the electrodes of the diagnosis device, if there is any deviation from the measurement carried out by the doctor and repeated at home, the characteristics of the boundary values are compared with one another and a warning signal is emitted if there is a discrepancy.

7. Device for carrying out the method according to claim 1, by means of an automatic ECG diagnosis device which has a measurement, control and diagnosis unit (2) suitable for storing a initial diagnosis produced in the surgery, the ECG diagnosis device having an analysis device (11) which compares the diagnosis made by the patient by self-measurement with the initial diagnosis and delivers a decision aid to the patient, **characterised in that** a checking unit for checking the sequence of discrepancies is attached to the diagnosis unit (2).

8. Device according to claim 7, **characterised in that** a delay unit (8) for the measurement initiation is attached the measurement, control and diagnosis unit (2).

9. Device according to claims 7 or 8, **characterised in that** a signal quality check unit (9) is attached to the diagnosis unit (2).

## Revendications

1. Procédé destiné à un auto-examen électrocardiologique, au moyen d'un dispositif de diagnostic à électrocardiogramme, équipé de moyens pour mémoriser au moins un diagnostic et pour générer une aide à la décision pour le patient, présentant les étapes suivantes :
- un diagnostic de base, caractéristique de l'état de base du patient, est mémorisé dans le dispositif de diagnostic ;
- l'ordre de succession des électrodes appliquées est contrôlé avec de procéder à une auto-mesure et un avertissement de défaut est émis en cas d'écart ;
- un diagnostic réel est établit d'après une auto-mesure du patient et mémorisé dans le dispositif;
- le diagnostic réel est comparé au diagnostic de base ;
- l'aide à la décision est mise en fonction en se basant sur la comparaison.

2. Procédé selon la revendication 1, **caractérisé en ce que** le diagnostic de base, caractéristique de l'état de base du patient et à comparer au diagnostic réel ultérieur, est un diagnostic établi antérieurement dans la pratique médicale au sujet du patient et mémorisé dans le dispositif.

3. Procédé selon les revendications 1 et 2, **caractérisé en ce que** le diagnostic de base, caractéristique de l'état de base du patient et à comparer au diagnostic réel ultérieur, est un diagnostic sélectionné dans le stock de diagnostics du dispositif de diagnostic, caractéristique de la maladie du patient et mémorisé dans le dispositif.

4. Procédé selon les revendications 1 à 3, **caractérisé en ce que**, après lancement du début de mesure à ECG par le patient, la fourniture automatique des données de mesure par le dispositif de diagnostic à ECG est mise en marche, de manière retardée.

5. Procédé selon les revendications 1 à 4, **caractérisé en ce que**, dans le cadre de l'auto-mesure par le patient, l'agencement correct des électrodes du dispositif de diagnostic à ECG sur le corps à examiner est vérifié par le contrôle de la qualité de signal du signal ECG, par le fait que la différence, mesurée dans les limites du domaine sans signal de la courbe, entre les amplitudes maximale et minimale survenant, est observée comme étant un bruit et, selon son rapport par rapport à une valeur seuil, le signal est accepté ou rejeté.

6. Procédé selon les revendications 1 à 5, **caractérisé en ce que**, dans le cadre de l'auto-mesure par le patient, pour le contrôle de l'ordre de succession correct des électrodes du dispositif de diagnostic, à chaque déduction de la mesure effectuée par le médecin et répétée à domicile, les types propres des valeurs aux limites sont comparés entre eux et un signal d'avertissement est fourni dans la cas où il y a un écart.

7. Dispositif de mise en oeuvre du procédé selon la revendication 1, à l'aide d'un dispositif de diagnostic à ECG automatique, comprenant une unité de mesure, de commande et de diagnostic (2), convenant pour mémoriser un diagnostic de base établi dans la pratique médicale, le dispositif de diagnostic à ECG présentant un appareil d'analyse (11), comparant au diagnostic de base le diagnostic induit par auto-mesure par le patient, et fournissant une aide à la décision au patient, **caractérisé en ce qu'**une unité de contrôle, pour contrôler l'ordre de succession des dérivations, est raccordée à l'unité de diagnostic (2).

8. Dispositif selon la revendication 7, **caractérisé en ce qu'**une unité de retardement (8) pour le début de la mesure est raccordée à l'unité de mesure, de commande et de diagnostic (2)

9. Dispositif selon la revendication 7 ou 8, **caractérisé en ce qu'**une unité de contrôle de qualité de signal (9) est raccordée à l'imité de diagnostic (2).
